Europäisches Patentamt

(19) European Patent Office

Office européen des brevets

(11) Publication number: **0 044 366**
**B1**

(12) **EUROPEAN PATENT SPECIFICATION**

(45) Date of publication of patent specification: **09.01.85**

(21) Application number: **80302490.0**

(22) Date of filing: **23.07.80**

(51) Int. Cl.⁴: **B 01 D 9/04** // B01D3/06, B01D9/00

(54) **Distillative freezing process for separating close boiling mixtures.**

(43) Date of publication of application:
27.01.82 Bulletin 82/04

(45) Publication of the grant of the patent:
09.01.85 Bulletin 85/02

(84) Designated Contracting States:
AT BE CH DE FR GB IT LI LU NL SE

(56) References cited:
EP-A-0 015 157
AU-B- 429 575
DE-B-1 292 635
US-A-2 777 299
US-A-3 298 796
US-A-3 558 731
US-A-3 690 116
US-A-4 003 213

(73) Proprietor: **Cheng, Chen-Yen**
**9605 La Playa Street, NE.**
**Albuquerque New Mexico, 87111 (US)**
(73) Proprietor: **Cheng, Sing-Wang**
**4th Floor, No.1, Lane 479**
**Fu-Hsing N.Road Taipei (TW)**

(72) Inventor: **Cheng, Chen-Yen**
**9605 La Playa Street, NE.**
**Albuquerque New Mexico, 87111 (US)**
Inventor: **Cheng, Sing-Wang**
**4th Floor, No.1, Lane 479**
**Fu-Hsing N.Road Taipei (TW)**

(74) Representative: **Wells, Keith Raymond et al**
**POTTS, KERR & CO. 15 Hamilton Square**
**Birkenhead Merseyside L41 6BR (GB)**

Courier Press, Leamington Spa, England.

## Description

The present invention relates to a distillative freezing process suitable for use in separating a mixture containing at least two volatile components.

The separation of a mixture containing volatile components is a very important operation in chemical industries. In some cases, the purity required in the products is very high. Examples of this are the production of polymerization grade monomers, such as ethylene, propylene, styrene, butadiene and p-xylene. Distillation operations have hitherto been used for such separation in most cases, although freezing operations have been used in some special cases.

When a distillation operation is used in separating a mixture containing components that are very close in boiling points and volatilities, the number of plates employed is very large and a large reflux ratio has to be used. Accordingly, both plant and operating costs are very high. Furthermore, these costs increase greatly as the required purity of the product increases. The separation of ethylene-ethane mixtures, propylene-propane mixtures, styrene-ethylbenzene mixtures and p-xylene-m-xylene mixtures for use in producing high grade ethylene, propylene, styrene and p-xylene respectively, are good examples. There is a great need for finding a better and more economical way of accomplishing the desired separations.

In a conventional fraction solidification process, a feed containing a crystallizing component and one or more impurities is brought into a two phase solid-liquid region to form a mixture containing crystals of the crystallizing component and a liquid mixture containing the impurities. An expensive scraped surface freezer is usually used in this operation. A centrifuge or a hydraulic washing column is then used to free the crystals from the mother liquor. Even though the crystals formed are usually very pure, it has been very difficult to produce a very high purity product of the crystallizing component, because a complete separation of the crystals from the surrounding impure liquid phase is difficult. Furthermore, the costs of the plant used in a conventional solidification process is usually very high.

It is important to distinguish the distillative freezing process of the present invention from a vacuum freezing desalination process, a vacuum crystallization process, a vacuum drying process, and a conventional desublimation process for removing a component from a gas mixture. In a vacuum freezing desalination process (also called an evaporative freezing process), only one component (water) vaporizes and the same component (water) freezes. In a vacuum crystallization process or a vacuum drying process, only one component (solvent) evaporates while the other component (solute) crystallizes. In a conventional desublimation process, a gas mixture is cooled down to a very low temperature to remove a crystallizing component (e.g. carbon dioxide or phthalic anhydride) by a desublimation operation and the residual gas is simply discharged. In a distillative freezing process, two or more components are vaporized from a feed under a sufficiently reduced pressure and only one component freezes. The low pressure vapor formed in a distillative freezing process is transformed completely into a condensed mass without pressurization by slightly lowering its temperature. The phase behaviour of a binary system to which a distillative freezing process applies is distinct from phase behaviours of binary systems to which these processes apply.

A process in accordance with the present invention is suitable for separating a mixture which contains at least two key components that are volatile and have close boiling temperatures and close volatilities. One of the components is a non-crystallizing component and is denoted as A-component and the other is a crystallizing component and is denoted a B-component.

According to the present invention there is provided a crystallization separation process for separating a multi-component mixture which is at least partly in a liquid state and includes two key volatile components denoted respectively as A-component and B-component, the A-component not being crystallized and whilst the B-component is, into a B-enriched portion and a B-lean portion through formation of a substantially pure B-solid mass, wherein the two key components form a binary system

(a) whose characteristic vapor pressure ratio defined as the ratio of the vapor pressure of A-component to that of B-component both evaluated at the triple point temperature of B-component is in the range of 0.1 to 10 and is less than the ratio of the heat of sublimation to the heat of melting of B-component evaluated at the triple point temperature of B-component, and

(b) whose constant pressure phase diagram includes a three phase (B-enriched solid, liquid and vapor) state, a two phase (B-enriched solid and vapor) region covering a substantial concentration range above the temperature of the three phase state and a two phase (B-enriched solid and liquid) region below the temperature the three phase state, comprising a first step of forming a vapor mixture and a first condensed mass formed of a mother liquor and a solid phase mass of substantially pure B-component by concurrently vaporizing the volatile components from the mixture in a first zone under a first temperature and a first pressure that are respectively lower than the triple point temperature and the triple point pressure of the pure B-component, the heat released in the formation of the B-enriched solid being at least partially removed by the concurrent vaporization of the two components from the mixture, and a second step of transforming, in a second zone, the vapor mixture into a second condensed mass which is at least partly in a liquid state by transferring heat therefrom while maintaining the vapor under a second pressure which is also lower than the triple point pressure of the B-component.

The solid phase is substantially pure B-component. Preferably, the first step is carried out under substantially adiabatic conditions. The temperature in the second step is higher than the eutectic temperature of the binary system at a low pressure.

In the process, the heat of crystallization is withdrawn at least partly by concurrently vaporizing portions of the two components from a mixture under a sufficiently reduced pressure that is lower than the triple point pressure of the B-component to form a low pressure vapor mixture and a slurry that comprises a mother liquor and a solid phase enriched with B-component. The B-enriched solid phase is separated from the mother liquor and melted to yield a B-enriched product.

In a first modified process, the concurrent vaporization step described is continued until the mother liquor is completely or substantially completely eliminated so that substantially all of the A-component is transferred to the low pressure vapor phase. For example, p-xylene of better than 99.98% purity has been obtained.

The low pressure vapor mixture obtained in each of these processes contains substantial amounts of both components. This vapor is transformed into a condensed mass, either a simple condensate (liquid) or a condensate-desublimate (liquid and solid) mixture, by a slight lowering in its temperature without a substantial pressurization. When the condensed mass is completely in the liquid state, a continuous process can be used in condensing the low pressure vapor. When the condensed mass is a condensate-desublimate mixture, the tendency for the desublimate to adhere on the cold surface is reduced by the presence of the condensate (liquid). Even when the desublimate formed does adhere on the cold surface, a simple method to be described can be used in dislodging it from the cold surface.

The features that both A-component and B-component are vaporized in the first characteristic step and that their volatillities are close lead to the following advantages:

(1) The concurrent vaporization of both components enables withdrawal of a large amount of heat and results in formation of a large amount of B-solid.
(2) The vapor formed is a mixture containing substantial amounts of both components and can be transformed completely into a condensed mass by a slight lowering in temperature without a substantial pressurization. This temperature is higher than the eutectic temperature of the binary system.
(3) The mother liquor phase can be completely eliminated by continuing the first characteristic step to yield an uncontaminated B-solid.

The invention will be further described, by way of example, with reference to the accompanying drawings, in which:—

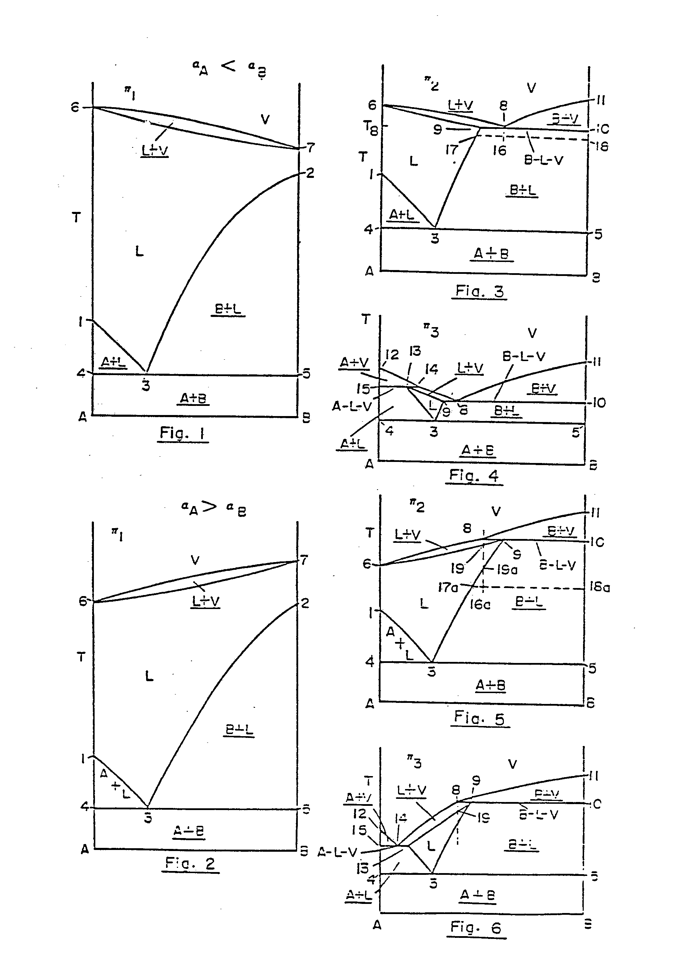

Fig. 1 illustrates a phase diagram of a first binary system containing two volatile components with close volatilities at a pressure higher than the triple point pressures of the components, in which the volatility ($\alpha_A$) of the first component (A-component) is less than that ($\alpha_B$) of the second component (B-component) and the melting temperature ($T_A$) of the first component is less than that of the second component ($T_B$).

Fig. 2 illustrates a similar phase diagram for a second binary system in which the volatility ($\alpha_A$) of the first component is higher than that ($\alpha_B$) of the second component.

Fig. 3 illustrates a phase diagram of the first binary system shown in Fig. 1, taken at a pressure lower than the triple point pressure of B-components, and shows the existence of a three phase state having B-solid, liquid and vapor, a two phase (B-solid and vapor) region above the three phase temperature and the existence of a two phase (B-solid and liquid) region below the three phase temperature.

Fig. 4 illustrates a phase diagram of the first binary system taken at a pressure substantially lower than that of Fig. 3, showing a three phase (A—L—V) state wherein A-solid and liquid and vapor coexist, a two phase (A+V) region wherein A-solid and vapor coexist and a two phase (A+L) region wherein A-solid and liquid coexist at the A-rich end.

Figs. 5 and 6 are similar to Figs. 3 and 4 respectively and are phase diagrams for the second binary system shown in Fig. 2, it being noted from Figs. 3 to 6 that the phase diagram taken at an operating pressure of a system to which a distillative freezing process applies has a three phase (solid-liquid-vapor) temperature, a two phase (solid and vapor) region above the three phase temperature and a two phase (solid and liquid) region below the three phase temperature.

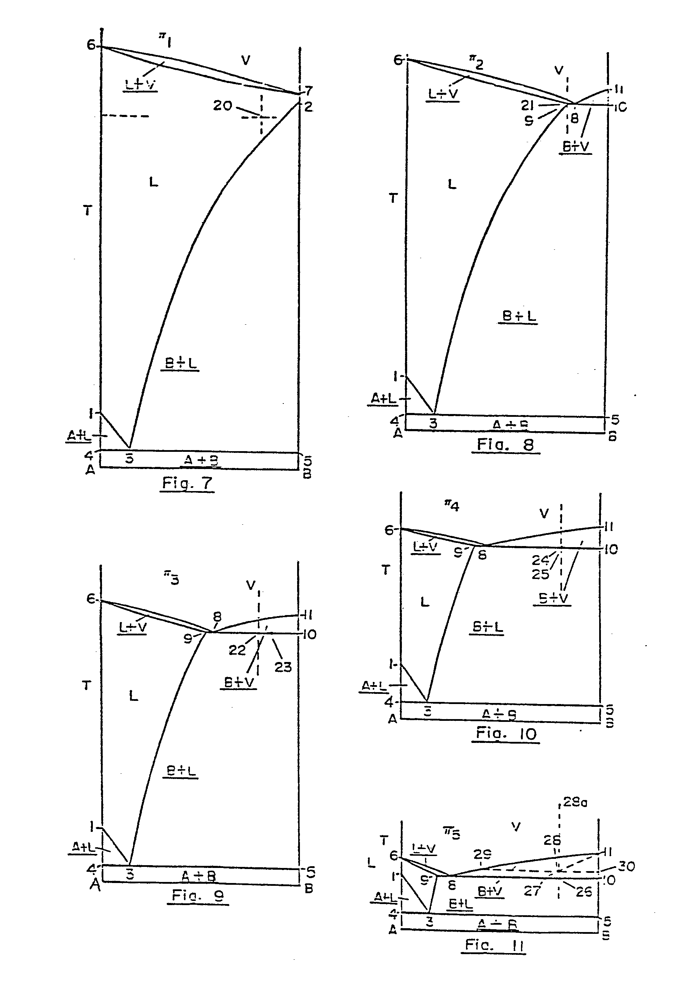

Figs. 7 to 11 illustrate how phase diagrams of a binary system containing two volatile components vary as the pressure is reduced and will be used in explaining the progress of a distillative freezing process.

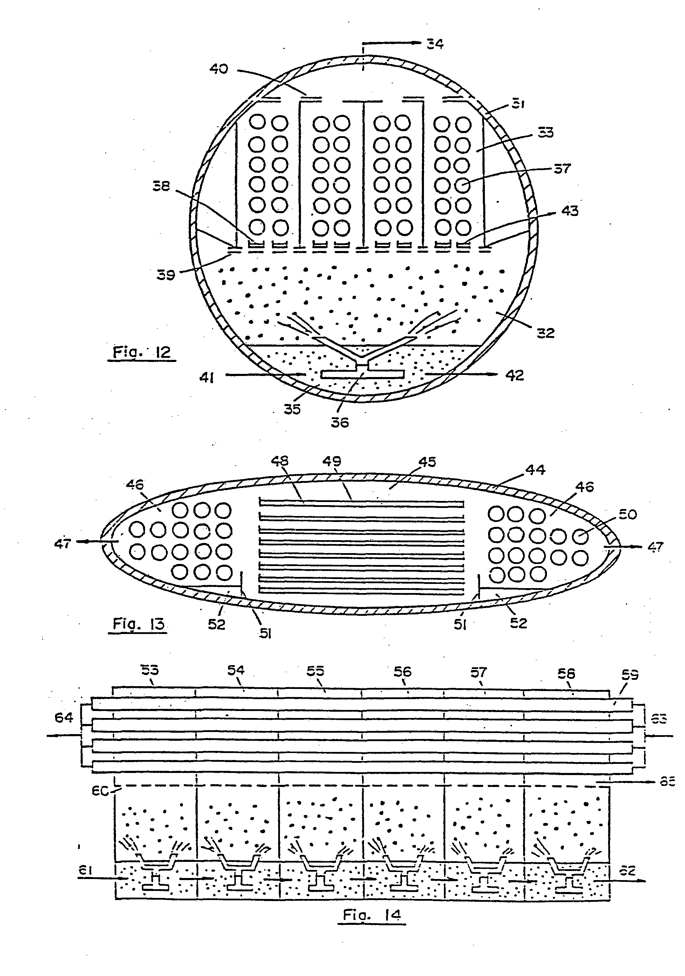

Figs. 12, 13 and 14 illustrate apparatuses that can be used in conducting the basic distillative freezing process of the present invention, each of these Figs. showing a first processing zone in which concurrent vaporizations of the two components and simultaneous crystallisation of B-solid take place and a second processing zone in which the low pressure vapor formed in the first zone is transformed into a condensed mass.

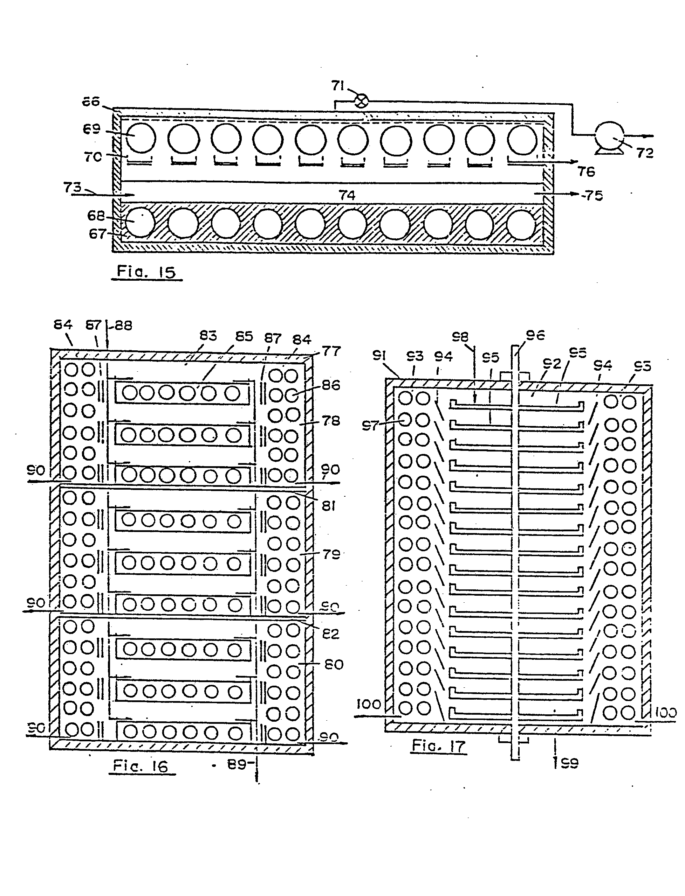

Figs. 15 to 17 illustrate apparatuses that can be used in conducting a first modified process in accordance with the present invention, each of these Figs. showing two processing zones, in the first of which concurrent vaporization of the two volatile components can be continued to completely eliminate the mother liquor phase.

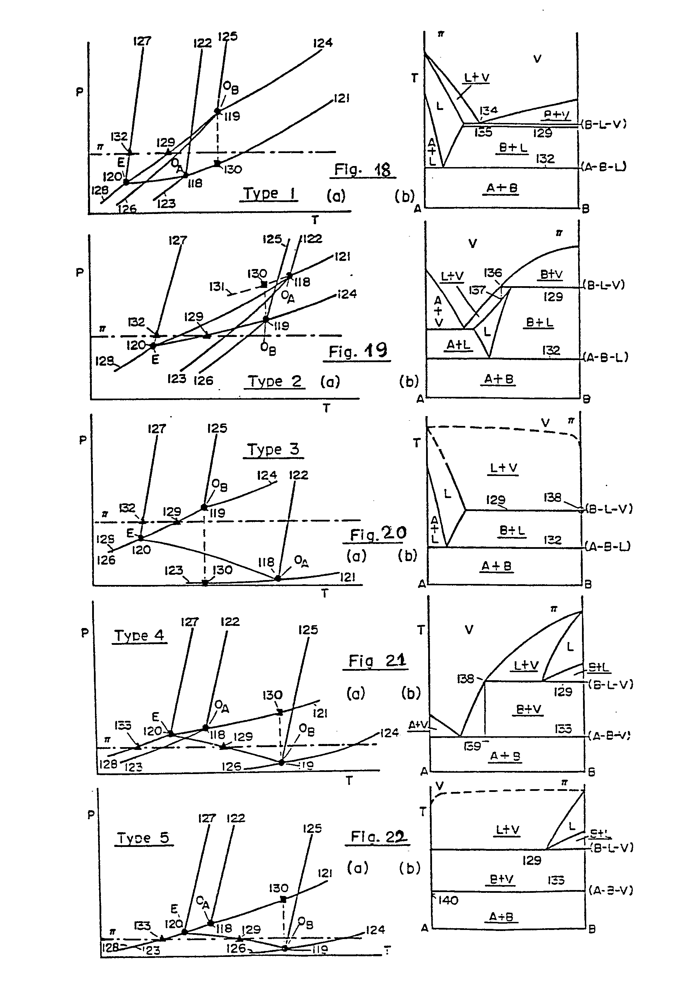

Figs. 18 to 22 will be used in distinguishing systems to which a process of the present invention can be applied from systems to which separation processes of the prior art, such as vacuum freezing process for desalination, a vacuum crystallization process, a vacuum drying process and a conventional desublimation process, can be applied.

The process or modified processes of the present invention have several basic unit operations. These unit operations can be well explained by referring to some phase diagrams.

The processes of the present invention can be used in separating a mixture containing at least two volatile components, irrespective of whether the two components form an azeotropic mixture or not. For simplicity, however, the following discussions will be limited to mixtures of components which do not form an azeotrope. However, the theory can be extended to, and used for, azeotrope-forming mixtures.

Figure 1 illustrates a typical phase diagram of a binary system having two volatile components (A and B) at a pressure $\pi_1$ higher than the triple point pressure of the components. The figure shows a first freezing line 1—3 (saturated with A), a second freezing line 2—3 (saturated with B), an eutectic line 4—3—5, one eutectic point 3, and liquid vapor lines 6—7. In the system illustrated, the volatility of B (denoted as $\alpha_B$) is higher than the volatility of A (denoted as $\alpha_A$). Fig. 2 illustrates a similar phase diagram for another typical binary system in which the volatility of A is higher than that of B. It is assumed that the volatilities of the two components are reasonably close to each other.

When a mixture of a composition in the range 3—5 is cooled and brought into the region 2—3—5, a part of B-component in the mixture crystallizes out. Therefore, for a mixture in the composition range 3—5, B-component is called the crystallizing component and A-component is called the non-crystallizing component. Conversely, for a mixture of a composition in the range 4—3, A-component and B-component are respectively the crystallizing component and the non-crystallizing component.

A binary mixture treated by the processes of the present invention is called either a Type 1 mixture or a Type 2 mixture according to whether the volatility of the crystallizing component is greater or less than the volatility of the non-crystallizing component. Therefore, mixtures in the range 3—5 of Figure 1 and mixtures in the range of 4—3 of Figure 2 are Type 1 mixtures; conversely, mixtures in the range 4—3 of Figure 1 and mixtures in the range 3—5 of Figure 2 are Type 2 mixtures. In the following sections, a mixture in the range 3—5 of Fig. 1 will be used to represent a Type 1 mixture and a mixture in the range 3—5 of Fig. 2 will be used to represent a Type 2 mixture.

The phase diagram of the system of Fig. 1 taken at a pressure $\pi_2$ which is lower than the triple point pressure of B but higher than that of A is shown in Fig. 3. In Fig. 3, one can see a B-solid-liquid-vapor line 9—8—10, denoted as a B—L—V three phase line, a B-solid-vapor region 8—10—11, denoted at (B+V) two phase region and a B-solid-liquid region 9—10—5—3, denoted as (B+L) two phase region. The presence of the three phase line indicates that the liquid phase 9, the vapor phase 8 and the B-solid phase 10 can reach a three phase equilibrium state under pressure $\pi_2$ and temperature $T_8$. The pressure and temperature will be respectively referred to as a B—L—V three phase pressure and a B—L—V three phase temperature. It is seen that the (B+V) region and the (B+L) region are respectively above and below the B—L—V temperature. The presence of the B-solid-vapor region indicates that when a mixture of a composition in the range 8—10 is brought to a state represented by a point in the (B+V) region, it separates into a B-solid mass and a vapor mixture represented by a point on the saturated vapor line 8—11. A liquid phase does not exist in this region. A corresponding phase diagram for the system of Fig. 2 is shown in Fig. 5. In this figure, one can also see the presence of a B—L—V three phase line 8—9—10, a B-solid-vapor region 8—10—11 above the B—L—V temperature and a B-solid-liquid region 9—10—5—3 below the B—L—V temperature. By comparing the two three phase lines in these two figures, one notices that, for the Type 1 system, the equilibrium vapor 8 is richer in B than the equilibrium liquid 9 and that for the Type 2 system the reverse is true.

Figure 4 shows the phase diagram of the system of Fig. 1 taken at a low pressure $\pi_3$ that is lower than the triple point pressure of A-component. In the figure one can see another three phase line 15—13—14, denoted as an A—L—V line, another solid-vapor region, denoted as (A+V) region, and another solid-liquid region, denoted as (A+L) region in addition to those seen in Fig. 3. Figure 6 shows a similar phase diagram for the system of Fig. 2. Again, one can see an A—L—V line 15—14—13, an (A+V) region and an (A+L) region. Descriptions similar to those given for the B—L—V lines, (B+V) regions and (B+L) regions can be given to these A—L—V lines, (A+V) regions and (A+L) regions.

Phase diagrams of systems to which processes of the present invention can be applied and phase diagrams of systems to which processes of the prior art can be applied will be compared hereinafter with reference to Figures 19 to 23. Characteristic features of systems to which the present processes can be applied will also be described hereinafter.

The process and modified processes of the present invention are closely related to the presence of the three phase lines and solid-vapor regions in the phase diagrams described. There are several key unit operations in these processes and important information about these unit operations can be obtained by studying these phase diagrams. These unit operations will now be described.

(1) Three phase transformation (denoted as B—L—V operation)

In a basic distillative freezing process, a mixture is transformed into a solid, a mother liquid and a vapor mixture. These coexisting phases can be closely represented by the three phases on a three phase line.

(2) Solid-Vapor region transformation (denoted as (B+V) operation)

In a modified distillative freezing process, a mixture is transformed to a B-solid and a low pressure vapor mixture. Such a state can be represented by a point in the solid-vapor region of a phase diagram. There is no liquid phase under such condition and there is no need to separate solid from mother liquor. Therefore, a modified distillative freezing process will also be referred to as a solid-vapor region refining process.

(3) A mixed condensation-desublimation operation

As has been described, a solid, a mother liquor and a low pressure vapor mixture are formed from a feed mixture in the basic distillative freezing process. In order to maintain the processing system under the reduced operating pressure, the low pressure vapor has to be transformed into a condensed mass. It is desirable to accomplish this without substantially pressurizing the vapor. Let points 9, 8, 10 in Fig. 3 respectively represnet the mother liquor phase, the vapor phase and the solid phase obtained in a three phase transformation of a Type 1 mixture. When the vapor 8 is cooled under the pressure $\pi_2$ to a temperature 16 which is slightly lower than the three phase temperature, it is transformed into a liquid (condensate) 17 and a solid (desublimate) 18. Therefore, this transformation is referred to as a mixed condensation-desublimation operation or, simply, a condensation-desublimation operation. It is noted that this operation is possible because the (B+V) region and the (B+L) region are respectively above and below the B—L—V temperature.

When a low pressure pure vapor is brought in contact with a cold surface so that the vapor desublimes the solid formed (desublimate) adheres to the cold surface and initiates heat transfer. Therefore, there is a need to remove the desublimate frequently. it is fortunate that in a process of the present invention the vapor formed is a mixture of A-component and B-component and a step of transforming the vapor to a condensed mass is either a mixed condensation-desublimation operation or a simple condensation operation. It is not a simple desublimation operation.

The desublimate formed in a condensation-desublimation operation in a process of the present invention has a much lower tendency to adhere on the cold surface. This is because the desublimate/condensate ratio obtained is usually low and the condensate formed tends to wet the cold surface and prevent the desublimate from adhering to the cold surface. Referring to Fig. 3, the desublimate/condensate ratio is given by the ratio of $\overline{16—17}$ to $\overline{16—18}$. When a mixture containing close boiling components is treated by a process of the present invention, the vapor phase 8 and the liquid phase 9 are very close in compositions. Provided that slight cooling is applied, points 16 and 17 are very close. Therefore for such a close boiling system, the desublimate/condensate ratio is very small. Accordingly, the closer the volatilities of the components in a mixture treated by a distillative freezing process causes the desublimate/condensate ratio obtained to be lower thereby making it easier to carry out the condensation-desublimation operation.

Put another way, the closeness of the volatilities of the components of a mixture makes a distillation separation process difficult but makes a distillative freezing separation process easier.

When a mixture is subjected to a solid-vapor region refining process (a modified distillative freezing process), the vapor formed can be represented by any point on the saturated vapor line 8—11. However, in order to obtain a high yield of the B-solid, it is desirable that the vapor obtained be represented by a point close to the three phase point 8. By so doing, the above reasoning can also be applied to the solid-vapor region refining process.

It will now be assumed that the vapor, the mothor liquor and the solid formed from a Type 2 mixture by a three phase transformation operation are represented by points 8, 9 and 10 in Fig. 5. When the vapor is cooled without being pressurized substantially, it condenses completely to form a condensate at a temperature of point 19. This operation is referred to as a simple condensation of the vapor mixture. That a simple condensation has taken place is due to the fact that the volatility of the non-crystallizing component $(\alpha_A)$ is higher than that of the crystallizing component $(\alpha_B)$ and because the (B+V) region and the (B+L) region are respectively above and below the B—L—V temperature.

It is noted however, that when the condensate 19 is cooled down further to 16a, the condensate does separate into a solid and a liquid and the ratio of the two phases is 16a—17a to 16a—18a. Letting, the intersection of line 19—16a and 3—9 be 19a, the range 19—19a is the range of undercooling in which a simple condensation can take place. When the volatilities of the two components are very close, the line segments 8—9, 8—19 and 19—19a are very short. Therefore, if the condenser temperature is not closely controlled, some solid deposition on the condenser wall will take place even though the mixture treated is a Type 2 mixture.

The three phase transformation step in a basic distillative freezing process may be conducted under an adiabatic condition. Accordingly, the latent heat released in forming B-solid is mostly removed by vaporizing the A-component and B-component. The fact that the non-crystallizing component is also volatile is helpful in the removal of heat of crystallization of B-solid.

Processing of a Type 1 mixture by the basic distillative freezing process comprises the following five steps:

(1) Step 1: Three phase transformation

In this step, a feed mixture is transformed into a low pressure vapor and a condensed mass which comprises a mother liquor and B-solid.

(2) Step 2: Purification of B-solid

In this step, the B-solid in the condensed mass obtained in Step 1 is separated from the mother liquor and purified. The separated mother liquor constitutes a B-lean product.

(3) Step 3: Condensation-desublimation

In this step, the low pressure vapor mixture obtained in Step 1 is cooled without being substantially pressurized and is transformed into a condensate-desublimate mixture.

(4) Step 4: Melting of purified B-solid

In this step, the purified B-solid obtained in Step 2 is melted to give a B-enriched product.

(5) Step 5: Melting of desublimate

In this step, the desublimate obtained in Step 3 is melted. The condensate-desublimate is transformed into a liquid mixture which may also become a B-lean product.

When a Type 2 mixture is processed, the processing steps taken are substantially the same as those indicated above except that Step 3 becomes a simple condensation operation and Step 5 may be eliminated. Each of the steps listed are explained in detail hereinafter.

The three phase transformation step may be conducted with heat addition, with heat removal, or under adiabatic conditions. When this step is conducted under substantially adiabatic conditions, the heat released in forming B-crystals is removed mainly by vaporizing portions of the A-component and B-component. The operation may be carried out either as a differential process or as an equilibrium process.

An equilibrium and adiabatic operation will be described by referring to Figures 7 through 11. The figures respectively show phase diagrams of a Type 1 system at successively lower pressures, $\pi_1$ through $\pi_5$. In order to describe the process in a less abstract manner, A and B will be assumed to be m-xylene and p-xylene respectively, and $\pi_1$, $\pi_2$, $\pi_3$, $\pi_4$, and $\pi_5$ are approximately 6 mm $H_g$, 2.4 mm $H_g$, 0.8 mm $H_g$, 0.34 mm $H_g$ and 0.05 mm $H_g$. There is no three phase line and no (B+V) region in Figure 7, since $\pi_1$ is higher than the triple point pressure of B. It is seen in the figures that the three phase line 9—8—10 increases its length and the (B+V) region 8—10—11 expands as the pressure decreases. A series of events take place which will now be described:—

(a) It will be assumed that the feed is introduced under a condition represented by point 20 in Figure 7. The feed is thus in a liquid state.

(b) Referring to Fig. 8, when the crystal pressure is reduced to $\pi_2$, the state of the mixture is represented by point 21 which coincides with the equilibrium liquid on the three phase line. A three phase transformation is initiated at this condition. A differential amount of vapour is formed and a differential amount of B-solid is formed.

(c) Referring to Fig. 9, when the system pressure is reduced to $\pi_3$, the state of the mixture is represented by point 22. Substantial amounts of B-solid and vapor have been formed by this time. The ratio of the mass of B-solid ($S_3$) to the mass of vapor ($V_3$) is about equal to the ratio of latent heat of vaporization ($\lambda_v$) to latent heat of crystallization ($\lambda_f$) of B-solid and is represented by $\overline{8—23}/\overline{23—10}$. One may write

$$\frac{S_3}{V_3} = \frac{\lambda_v}{\lambda_f} = \frac{\overline{8—23}}{\overline{23—10}} \qquad (1)$$

The mother liquor mass ($L_3$) that remains is related to the sum of solid and vapor masses ($S_3+V_3$) by

$$\frac{L_3}{S_3+V_3} = \frac{\overline{22—23}}{\overline{9—22}} \qquad (2)$$

One may determine the masses of B-solid, mother liquor and vapor by using the above equations.

(d) Referring to Fig. 10, when the system pressure is reduced to $\pi_4$, the state of the mixture is represented by point 24. Let point 25 be such that the ratio 8—25 to 25—10 is equal to $\lambda_v/\lambda_f$. It is seen that point 24 coincides with point 25 at this pressure. Since the mother liquor mass (L₄) is related to the sum of solid and vapour masses ($S_4+V_4$) by

$$\frac{L_4}{S_4+V_4} = \frac{\overline{24-25}}{\overline{9-25}} \quad (3)$$

and since 24—25 is zero, the mass of mother liquor is zero.

(e) Referring to Fig. 11, when the system pressure is further reduced to $\pi_5$, the state representing the mixture is represented by a point 28 within the (B+V) region. The mixture is separated into B-solid and vapor and the ratio of the masses $S_5/V_5$ is given by 29—28/28—30.

For the given feed, a three phase transformation has to be conducted under a pressure between $\pi_2$ to $\pi_4$ and a solid-vapor region transformation step has to be conducted at a pressure lower than $\pi_4$.

When a mixture is subjected to an operation in which the vapor formed is removed as it is formed, the operation approaches a differential operation. A differential operation may be approximated by a number of equilibrium stage operations. As described above, when a given feed mixture is subjected to an equilibrium and adiabtic three phase transformation operation, the ratio of solid mass to vapor mass is substantially a definite value and the ratio of liquid mass to the combined mass of solid and vapor is a function of pressure. These ratios can be varied, however, by addition or removal of heat. It may therefore be desirable to conduct this operation under a non-adiabatic condition in order to control those ratios. Descriptions of a three phase transformation conducted under a non-adiabatic and/or differential condition can be arrived at by modifying the description given above for an equilibrium and adiabatic operation.

Figures 12, 13 and 14 illustrate apparatus in which the basic distillative freezing process can be conducted. The apparatus shown in Fig. 12 has an insulated enclosure 31 and consists of a first processing zone 32 in which a three phase transformation operation is conducted and a second processing zone 33 in which a condensation step or a condensation-desublimation step is conducted. The unit is connected to a vacuum pump through a conduit 34. A slurry pool 35 is present in the first zone and a spraying device 36 is used to spray the slurry into space so that the volatile components are vaporized from the droplets to form B-crystals within the droplets. The droplets fall on the pool. The second processing zone may or may not be compartmentized and contains working medium conduits 37. A working medium is contained in the conduits. There are troughs 38 placed under the working medium conduits and valving means 39, 40 are provided for each compartment.

A feed 41 is introduced into the first zone and is transformed into a low pressure vapor stream and a slurry that comprises a mother liquor and B-solid. The slurry stream 42 is removed from the unit and is separated into a mother liquor and purified B-solid. The mother liquor constitutes a B-lean product. The purified B-solid is melted and becomes a B-enriched product.

The low pressure vapor obtained in the first zone is admitted to the second zone and brought into a heat exchange relation with the working medium (a cooling medium) and is transformed into a condensed mass. The condensed mass may be completely in a liquid state or may be partly liquid and partly solid. In the latter case, the solid may or may not adhere to the conduit walls to interfere with heat transfer. When a solid does adhere to the conduit walls, there is a need to remove it from the walls intermittently. It is therefore desirable to prevent an adhering solid phase from forming. However, when an adhering solid phase does form, one has to have a convenient way of removing it.

When a type 1 mixture is processed, the condensed mass is a condensate-desublimate mix and is partly solid and partly liquid. When the conduit walls are properly wetted and the desublimate/condensate ratio is low, the desublimate may not adhere to the walls. It is then possible to conduct the condensation-desublimation operation in a continuous manner. In order to prevent desublimate from adhering on the walls, one may recycle some liquid and apply it on the walls. It has been described that when a type 2 mixture is processed and when the cooling operation is properly controlled, the condensed mass is completely in liquid state. In this case, a simple condensation operation has taken place and the operation can be conducted in a continuous manner. Provided that an adhering solid phase does not form, the second processing zone does not have to be compartmentized and the valving means 39, 40 shown are not needed.

However, when an adhering solid phase does form on the walls, it has to be dislodged from the walls, at least intermittently. The second processing zone is compartmentized and valving means are provided in each compartment for the purpose of melting at least a part of the adhering solid and dislodging it from the walls. While this operation is in progress in a compartment, the compartment is pressure isolated from the first zone and the vacuum conduit and a heating medium is introduced into the medium conduits. As this process takes place, the pressure in the compartment increases. The pressure increase is accomplished by vaporizing a small amount of the condensed mass in the compartment. The compartment pressure increases because there are solid, liquid and vapor existing in the compartment. As the adhering B-solid is melted, the liquid phase becomes richer in B-component. Under the solid-vapor-liquid equilibrium conditions, the system pressure and the liquid composition are related. It can be seen from the way compositions of liquids 9 vary in Figs. 8 through 11 that the system pressure increases as the liquid phase becomes richer in B-component. Therefore the compartment pressure must rise as adhering B-solid is melted.

The processing unit illustrated by Fig. 13 can be used when a mixture is processed in such a way that no solid phase adheres to the cold surface. It has an insulated enclosure 44 and has a first processing zone 45, and two second processing zones 46. Conduits 47 are provided in the second processing zones to evacuate the unit. Vaporization trays 48 containing liquid 49 are installed in the first zone and working medium conduits 50 are provided in the second processing zone. Baffles 51 are provided to provide a storage for a condensate or condensate-desublimate. In operation, a feed is added to the trays and the system pressure is reduced. The feed is transformed into a low pressure vapor mixture and a slurry. The slurry is removed from the trays, separated into a purified B-solid and a mother liquor. The melt of the purified B-solid and the mother liquor respectively become a B-enriched product and a B-lean product. The low pressure vapor is either transformed into a condensate or a condensate-desublimate and removed 52 from the system to become a B-lean product.

Figure 14 illustrates a system in which a multi-stage operation can be conducted. The unit illustrated has six sub-units 53 to 58 each having a first zone and a second zone. Feed 61 is introduced into the first zone of the first unit and transformed into a first vapor and a first slurry. The first slurry is transformed into a second vapor and a second slurry in the second sub-unit, etc. The sixth vapor and the sixth slurry 62 are formed in the sixth sub-unit 58. The sixth slurry is separated into a purified B-solid and mother liquor. A cooling medium 63 is introduced into medium conduits 59 that pass through the second zones of the sub-units and is discharged 64 from the left (as shown) end. The six vapor streams are transformed into condensates or condensates-desublimates which are discharged at the right end 65.

The first solid-vapor region refining process is a modified distillative freezing process. In this process, one feeds a liquid mixture $L_1$ (A+B) containing volatile components or a solid-liquid mixture which contains a liquid mixture and some solid of the crystallizing component $S(B)+L_1(A+B)$. It is noted that, in processing a feed containing a high concentration of contaminant A, it is advantageous to first apply a fractional solidification process to obtain a mixture enriched with B and subject the B-enriched mixture to the solid vapor region refining process. The feed then subjected to treatment is a mixture of B-solid and liquid $L_1(A+B)$ retained by the B-solid. It has been described that one way to transform a mixture into a B-solid and a vapor is to continue a three phase transformation step until the mother liquor phase is completely eliminated. It has also been described that it may be convenient to conduct a three phase transformation operation under adiabatic conditions. It is noted that the ratio of the B-solid mass and the vapor mass obtainable in a solid-vapor region transformation is a function of the feed concentration, the operating pressure, and the operating temperature. In order to maintain a given set of operating conditions, there is a need for heat addition or heat removal to satisfy the energy balance equation.

Processing of a Type 1 mixture or a solid-liquid mixture containing a Type 1 liquid comprises the following four steps:

(1) Step 1: Solid-vapor region transformation

In this step, a feed mixture is transformed into a low pressure vapor and a purified B-solid.

(2) Step 2: Condensation-desublimation

In this step, the low pressure vapor mixture obtained in Step 1 is cooled without being substantially pressurized and is transformed into a condensate-desublimate. Some of the desublimate formed may adhere to the cold surface.

(3) Step 3: Melting of purified B-solid

In this step, the purified B-solid obtained in Step 1 is melted to give a very pure B-product.

(4) Step 4: Melting of desublimate or adhering solid

In this step, the desublimate or adhering solid obtained in Step 2, is melted. The condensate-desublimate is transfromed into a liquid mixture which constitutes a B-lean product.

When a type 2 mixture is processed, step 2 may become a simple condensation step and step 4 is no longer needed. It is to be noted that steps 2 and 4 of this process are respectively similar to steps 3 and 5 of the basic distillative freezing processes. Therefore, descriptions of these steps can be omitted and only a description of Steps 1 and 3 are given in some detail in the following paragraphs.

In a solid-vapor region transformation, one may select both the pressure P and the Temperature T. Knowing the feed composition and temperature, and having selected the operating temperature and pressure, the yield and the required heat input or extraction in an equilibrium stage operation can be determined in the following manner. For example, let the feed be represented by point 20 in Fig. 7 and let the operation presure be $\pi_5$. On the phase diagram, a constant temperature line at the operating temperature is drawn, its intersection with the saturated vapor line 8—11 being referenced 29 and its intersection with the B-line being referenced 30. A constant composition line is then drawn through point 20 and itscintersections with the three phase line at $\pi_5$ and the

constant temperature line 29—30 are denoted by points 26 and 28 respectively. If the feed is $L_1$, the B-solid formed is S, and the vapor formed is $V_2$, the following relationships are obtained:—

$$\frac{S}{L_1} = \frac{\overline{29-28}}{\overline{29-30}} \quad (4)$$

$$\frac{V_2}{L_1} = \frac{\overline{28-30}}{\overline{29-30}} \quad (5)$$

$$\frac{S}{V_2} = \frac{\overline{29-28}}{\overline{28-30}} \quad (6)$$

The heat input required $Q/L_1$ per unit mass of feed is given by

$$\frac{Q}{L_1} = \frac{V_2}{L_1}(H_2-h_1)\frac{S}{L_1}(h_1-h_s) \quad (7)$$

where $H_2$, $h_s$ and $h_1$ are enthalpies per unit mass of the vapor, B-solid and feed respectively. By referring to equation (4) and to Fig. 11, it is to be seen that yield of B-solid increases as the operating temperature is brought closer to the three phase temperature.

The normal procedure to follow in the selecting the operating conditions for a solid-vapor region transformation are to select operating pressure from the cooling medium temperature to be used in transforming the low pressure vapor into a condensed mass, to select the temperature as the temperature slightly higher than the three phase temperature at the pressure, and to calculate the amount of heat to be supplied or removed by using equation 7.

When the mixture is subjected to an operation in which the vapor is removed as it is formed, the operation approaches a differential operation. The mixture first undergoes a differential B—L—V three phase transformation until the residual liquid is completely eliminated. Both the composition of the vapor removed and the composition of the residual liquid change with time. A differential operation may be approximated by a number of equilibrium stage operations.

While melting of purified B-solid takes place, the pressure within the zone has to be raised to a pressure together than the triple point pressure of B-component. It will be shown that, by pressure isolating the zone and by introducing a heating medium, a small amount of B-component vaporizes and produces the desired pressure increase. Melting then takes place in the zone. No compressor is needed in this operation.

Figure 15 illustrates an apparatus in which a solid-vapor region process can be conducted. The apparatus comprises an insulated enclosure 66, a heat conducting wall 67 enclosing a plurality of conduits 68 which contain a heat transfer medium, heat transfer tubes 69 containing a heat transfer medium, sliding valve means 70, and an evacuation conduit with a valve 71 connecting the enclosure to a vacuum pump 72. The enclosure is separated by the sliding valve menas, into a first processing zone which is located below the valve means and a second processing zone which is located above the valve means. Step 1 and Step 3 are conducted in the first zone and Step 2 and Step 4 are conducted in the second zone.

The operational procedure will be described with reference to the processing of a Type 1 binary liquid mixture in which the volatility of the crystallizing component is higher than that of the non-crystallizing component. Feed $L_1(A+B)$ 73 is introduced into the enclosure to form a layer of the mixture 74. If desired or necessary, a heating or cooling medium is passed through the conduits to maintain the mixture at the desired operating condition. A cooling medium is passed through the conduits 69 and the vacuum pump is actuated. A low pressure vapor $V_2(A+B)$ is formed, leaves the first zone and enters the second zone wherein it is condensed and desublimed into a mixture $M_2(A+B)$ containing B-solid and a liquid mixture. As the low pressure vapor leaves the mixture in the first zone, B-solid beings to form. In other words, the liquid mixture is transformed into B-solid and the low pressure vapor. The operations in the two zones are continued until the liquid phase in the first zone is substantially completely eliminated. It will be seen that, during this period, Step 1 and Step 2 are simultaneously conducted in the two zones. The sliding valve means 70 and the valve provided on the evacuation conduit 71 are then closed so that two zones are isolated from the vacuum line and are isolated from one another. Heating medium is then passed through conduits 68 and 69 to cause melting of the B-solid in the two zones. It will be seen that, during this period, Steps 3 and 4 are respectively conducted in the two zones and the pressures in these two zones are raised by vaporizing small amounts of the components in these zones. The pressure in the first zone is higher than the triple point pressure of B-component, and the pressure increase is due mainly to vaporization of B-component. The purified B-liquid that is formed in the first zone and the B-line liquid that is formed in the second zone are removed from the two zones to become two products L(B) and $L_2(A+B)$ respectively. The operations described complete a cycle and the next cycle is then initiated.

When a mixture processed is a Type 2 mixture in which the volatility of the crystallizing component is less than that of the non-crystallizing component, there may be no need for Step 4. Accordingly Step 3 is being conducted in the first zone, no process step is being carried out in the second zone.

The apparatus of Fig. 15 may also be used to process a feed that already contains B-solid. One simply introduces a mixture of B-solid and liquid instead of a liquid feed. The other operational steps are the same. One may also introduce a liquid feed $L_0(A+B)$ and transform it into a mixture of B-solid and a liquid mixture, prior to initiating the operating procedures for the solid-vapor region refining process. In carrying out this extra step, the sliding valve is closed to isolate the first zone from the second zone a feed is introduced into the first zone and a cooling medium is introduced into conduits 68 to remove heat from the mixture and to form B-solid therein. The procedures described are then followed.

The apparatus shown in Fig. 16 comprises an insulated enclosure 77 which is divided into three sub-units 78, 79, 80 by partitions 81, 82. Each sub-unit has a first processing zone 83 at its center and two second processing zones 84 on either side thereof. A plurality of heat conductive plates 85 containing conduits for working media are provided and heat transfer tubes 86 are provided in the second zones. The two zones are separated by sliding valve means 87. In operation, a feed mixture 88 is introduced to the heat conductive plates 85 and is cooled to the desired temperature. On evacuating the unit, a low pressure vapor and B-crystals are formed from the feed. This operation is continued until the liquid phase is completely eliminated. The low pressure vapor is admitted to the second zone through the sliding valve means 87 and is either condensed, or condensed and desublimed. When these operations are completed, these zones are isolated from one another by closing the sliding valve means and are isolated from the vacuum pump. Heating media are introduced into the conduits in the first zone and the heat transfer tubes in the second zone to melt the B-solid and desublimate in the two zones respectively. Again, pressures in these two zones are raised during this period and the pressure increases are caused by pressure isolation of the zones and vaporization of one or more components in the zones. The melt of the B-solid 89 and the liquid formed in the second zone 90 respectively become a purified B-product and a B-lean product.

Fig. 17 illustrates an apparatus in which a solid-vapor region transformation operation can be conducted continuously, the apparatus being somewhat similar to a rotary disk dryer. The apparatus has an insulated enclosure 91 having a first zone 92 and a second zone 93. The two zones are separated by louvered partitions 94. Rotating disks 95 attached to a rotating shaft 96 are provided in the first zone and heat transfer tubes 97 are provided in the second zone. In operation, a feed 98 is fed onto the first disk, the content of each disk being continually transferred to the next lower disk. As the feed moves downwards through the unit, a low pressure vapor is formed and the vapor is transformed to a condensed mass in the second zone. Purified B-solid 99 is removed from the bottom of the unit. The condensed mass 100 obtained in the second zone is removed at the bottom of the unit. When it is desired to supply or remove heat from the contents on the trays, heat transfer conduits located at the bottom of these trays may be employed.

One may also conduct a solid-vapor region transformation in a spraying system. The system comprises a spraying zone comprises an empty space and a spraying device, and a condensation or condensation-desublimation zone which includes heat transfer tubes containing a cooling medium. In operation, a feed either a liquid mixture $L_1(A+B)$ or a mixture containing a liquid mixture and some B-solid $S(B)+L_1(A+B)$ is sprayed to form small droplets in the spray processing zone, which zone is maintained at a low pressure by the cooling in the condenser or condenser/desublimer and a vacuum pump. The liquid mixture is converted into a low pressure vapor $V_1(A+B)$ and B-solid, and the B-solid is collected at the bottom of the zone. The B-solid is transferred through a conveyor into a mixer, wherein it is mixed with a recycled B-liquid stream to become a slurry. The slurry is transferred to a melter and is converted into pure B-liquid. A part of this liquid is discharged from the unit as a purified B-product and the remainder is recycled to the mixer. The low pressure vapor is converted into a condensed mass in the condenser or condenser-desublimer. When the condensed mass contains some B-solid, an additional step is needed to melt the B-solid. This may be done in the manner described with reference to the second zone shown in Fig. 15. It is to be noted that by virtue of the nature of the spray processing operation the solid-vapor region transformation is conducted in an adiabatic manner.

Figures 18a to 22a show P—T projections of P—T—C space phase models of Type 1 to 5 systems respectively. Each figure shows the triple point of A as 118 (denoted as $0_A$ point) the triple point of B as 119 (denoted as $0_B$ point), the vaporization lines of A and B as 118—121 and 119—124 respectively, the melting lines of A and B as 118—122 and 119—125 respectively, the four phase (A-enriched solid, B-enriched solid, liquid and vapor) point 120 (denoted as E-point or A—B—L—V four phase point); the three phase (A-enriched solid, liquid and vapor) line 118—120 (denoted as A—L—V line), the three phase (B-enriched solid, liquid and vapor) line 119—120 (denoted as B—L—V line), the three phase (A-enriched solid, B-enriched solid and liquid) line 120—127 (denoted as A—B—L line) and the three phase (A-enriched solid, B-enriched solid, and vapor) line 120—128 (denoted as A—B—V line).

It has been found convenient to use the terms "the characteristic vapor pressure ratio of a system" and "the characteristic latent heat ratio of a system" in the following discussions. The characteristic vapor pressure ratio of a system is defined as being the ratio of the vapor pressure of the non-crystallizing component to that of the crystallizing component, both being evaluated at the triple point temperature of the crystallizing component. The characteristic vapor pressure ratios of the systems illustrated are the ratios of the pressures at points 130 to the pressures at points 119 in the figures. Referring to Fig. 20*a*, when the $O_A$ temperature is higher than the $O_B$ temperature, the vaporization line of A-component has to be extended below the $O_A$ temperature to evaluate the vapore pressure of A-component at the $O_B$ temperature. The characteristic latent heat ratio is defined as the ratio of the latent heat of sublimation and the latent heat of melting of B-component evaluated at the $O_B$ temperature. For example, the characteristic latent heat ratios for water, benzene, and p-xylene are about 8.5, 3.48 and 4.44 respectively.

Certain comparisons can be drawn from Figures 18*a* to 22*a*. These are:—

(i) The component volatilities and the characteristic vapor pressure ratios

In a Type 1 system, both components are volatile but the volatility of the crystallizing component is greater than that of the non-crystallizing component. The characteristic vapor pressure ratio is less than one but is not near zero.

In a Type 2 system, both components are again volatile, the volatility of the crystallizing component being less than that of the non-crystallizing component. The characteristic vapor pressure ratio is greater than one but is still relatively small and is approximately equal to or less than the characteristic latent heat ratio.

In a Type 3 system, the non-crystallizing component is substantially non-volatile. Accordingly, the characteristic vapor pressure ratio is nearly equal to zero.

In a Type 4 system, both components are volatile and the volatility of the non-crystallizing component is much greater than that of the crystallizing component. The characteristic vapor pressure ratio is rather large and is greater than the characteristic latent heat ratio.

In a Type 5 system, the crystallizing component is substantially non-volatile. The characteristic vapor pressure ratio is extremely large and is much greater than the characteristic latent heat ratio.

(ii) The P—T slopes of the B—L—V lines

The P—T slopes of the B—L—V lines 119—120 for Type 1, Type 2 and Type 3 systems are positive, at least near the $O_B$ points. However, the P—T slopes of the B—L—V lines for the Type 4 and Type 5 systems are negative, at least near the $O_B$ points.

By effecting a thermodynamic analysis, one can derive an equation relating pressure to temperature of the B—L—V line. For ideal systems in which one may assume that (i) the ideal solution rule (Lewis and Randal Rule) applies to the liquid phase (ii) the ideal gas law applies to the gas phase and (iii) solubility of A-component in B-solid is negligible, one may arrive at the following conclusions:

(a) the P—T slope of the B—L—V line near the $O_B$ temperature is positive when the characteristic vapor pressure ratio is less than the characteristic latent heat ratio; and

(b) the P—T slope of the B—L—V line near the $O_B$ temperature is negative when the characteristic vapor pressure ratio is greater than the characteristic latent heat ratio.

For real systems, the conclusions derived may have to be slightly modified to allow for deviations from ideal behaviour. For instance, a system in which the characteristic vapor pressure ratio is slightly greater than the characteristic latent heat ratio may have a B—L—V line that has a positive P—T slope. Conversely, a system in which the characteristic vapor pressure ratio is slightly less than the characteristic latent heat ratio may have a B—L—V line which has a negative P—T slope. Bearing in mind that adjustments are required to allow for departures from the assumptions made in deriving the P—T sloep of a B—L—V line, the conclusions presented are very useful in determining what type of a system a mixture belongs to and whether a mixture can be successfully processed by a process of the present invention. Noting that the characteristic latent heat ratio of a substance is generally less than 10, it may be stated that the characteristic vapor pressure ratio of a mixture which can be processed by a process of the present invention is in the range of 0.1 to 10.

(iii) Inter-sections of constant pressure cross-sections with three phase lines

Referring to Figures 18*a* to 20*a*, it may be seen that the constant pressure cross-section of a P—T—C phase model which belongs to Type 1, Type 2 or Type 3 system when taken at a pressure on the B—L—V line (pressure at point 129) intersects with the A—B—L line (at point 132). Referring to Figures 22*a* and 22*b*, it may be seen that similar cross-section of a P—T—C phase model which belongs to Type 4 or Type 5 system intersects with the A—B—V line (at point 133).

Figure 18*b* illustrates a constant pressure cross-section of the P—T—C space phase model of a Type 1 system illustrated by Figure 18*a* taken at a pressure which intersects with the B—L—V line at point 129. Such a pressure is denoted as a B—L—V three phase pressure. Figures 19*b* to 22*b* illustrate similar cross-sections for the Type 2 to 5 systems shown in Figures 19a to 22a respectively. Pertinent conclusions which can be drawn from Figures 19b to 23b are:—

(iv) The (B—L—V) three phase temperature and the (B+V) two phase region.

In each of the Figures 18a to 22a there exists a B—L—V three phase temperature 129 at which B-solid, liquid and vapor coexist, and there exists a (B+V) region, a (B+L) region and a (L+V) region around the three phase temperature. Special attention is drawn to the size of the (B+V) region, the position of the (B+V) region relative to the (B—L—V) temperature and the position of the (B+L) region relative to the (B—L—V) temperature in each Figure.

Referring to Fig. 18b, it can be seen that in a Type 1 system a substantial (B+V) regions exists above the (B—L—V) temperature 129 and a (B+L) region exists below the (B—L—V) temperature 129. Because of the locations of the (B+V) and (B+L) regions relative to the (B—L—V) temperature, a vapor 134 formed in a B—L—V three phase transformation can be transformed into a condensed mass completely without a substantial pressurization by a small drop in temperature (to point 135) in a simultaneous condensation-desublimation operation. Because the (B+V) region covers a wide concentration range, a feed mixture within a wide concentration range can be processed by the basic distillative freezing process and the solid-vapor region refining processes of the present invention.

Referring to Fig. 19b, it can be seen that, in a Type 2 system, a substantial (B+V) region exists above the (B—L—V) temperature 129 and an (L+V) region and a (B+L) region exists below the (B—L—V) temperature 129. Because of the locations of the (B+V) region and the (L+V) region relative to the (B—L—V) temperature, a vapor 136 formed in a B—L—V three phase transformation can be transformed into a condensed mass completely without a substantial pressurization by a small drop in temperature (to point 137) in a simple condensation operation. Again, because the (B+V) region covers a wide concentration range, a feed mixture within a wide concentration range can be processed by a process of the present invention.

Referring to Fig. 20b, it can be seen that, in a Type 3 system, the two phase (B+V) region 138 is practically non-existent in the phase diagram. Accordingly, when a type 3 mixture is subjected to simultaneous flash vaporization and formation of B-solid, the vapor formed is substantially pure B-component and is desublime on cooling. Because a two phase (B+V) region does not exist in the phase diagram a Type 3 mixture can not be processed by a solid-vapor region refining operation.

Referring to Fig. 21b, it can be seen that, in a Type 4 system, a large two phase (B+V) region exists below the (B—L—V) temperature 129 and a two phase (B+L) region and a two phase (L+V) region exist above the three phase (B—L—V) temperature. Because the (B+V) region exists below the (B—L—V) temperature, a vapor formed by a three phase B—L—V transformation has to be cooled to a very low temperature 139 which is lower than the three phase (A—B—V) temperature 133 which, in turn, is lower than the four phase (A—B—L—V) temperature 120. A Type 4 gas mixture can be processed by a conventional desublimation process for recovering the crystallizing component. In such a process, no attempt has been made to transform the residual gas to a completely condensed mass.

Referring to Fig. 22b, it can be seen that the phase diagram of a Type 5 system is simlar to that of a Type 4 system except that a saturated vapor is pure A component. A Type 5 mixture can be processed by a vacuum crystallization and drying process. The vapor formed is substantially pure A-component and is usually either compressed and condensed, or compressed and discharged. In order to transform the vapor into a completely condensed mass without substantial pressurization, it must be cooled to a very low temperature 140 which is lower than the three phase (A—B—V) temperature 133 which in turn, is lower than the four phase temperature 120.

A process in accordance with the present invention can therefore be used for separating a mixture which belongs to a system having a substantial two phase (B+V) region above the three phase (B—L—V) temperature under a three phase (B—L—V) pressure.

In the solid-vapor region refining process, a mixture is brought into this two phase (B+V) region by completely eliminating the mother liquor phase. The vapor formed in a three phase (B—L—V) transformation or a solid-vapor region refining operation is transformed into a condensed mass by a small drop in temperature without a substantial pressurization. The temperature at which this operation is conducted is higher than the A—B—L—V four phase temperature and is higher than the normal eutectic temperature of the system.

A process in accordance with the present invention transforms a mixture into a low pressure vapor and solid crystallizing component and then transforms the low pressure vapor into a condensed mass. The operating pressures and operating temperatures of these steps are generally lower than the triple point pressure and temperature of the crystallizing component Table 1 lists triple point temperatures and pressures of various substances. The values listed are estimated from vapor pressure values listed in "Handbook of Chemistry and Physics", by The Chemical Rubber Company.

**0 044 366**

TABLE 1

Classification of substances according to triple point pressure

Class 1: Triple point pressure greater than 10 mm Hg

| Substance | Triple point temperature (°C) | Triple point pressure (mm Hg) |
|---|---|---|
| 1. Aluminum chloride | 192.4 | 1800 |
| 2. Ferric chloride | 304 | 480 |
| 3. Hydrogen cyanide | −13.2 | 135 |
| 4. Hydrogen chloride | −114.3 | 100 |
| 5. Ammonia | −77 | 50 |
| 6. tert-Butyl alcohol | 25.3 | 42.2 |
| 7. Hydrogen | −259.1 | 40 |
| 8. Cyclobutane | −50.0 | 39.2 |
| 9. Benzene | 5.5 | 35.85 |
| 10. 1,2,4,5 Tetrachlorobenzene | 139 | 31.1 |
| 11. tert-Butyl chloride | −26.5 | 26.25 |
| 12. 1,4 Dioxane | 10 | 18.2 |
| 13. Tetrachloroethylene | −35 | 14.34 |
| 14. Chlorine | −100.7 | 10 |
| 15. Chlorine dioxide | −59.0 | 10 |

Class 2: Triple point pressure in the range of 1 mm Hg to 10 mm Hg

| Substance | Triple point temperature (°C) | Triple point pressure (mm Hg) |
|---|---|---|
| 1. Acetic acid | 16.7 | 9.5 |
| 2. 1,4 Dichlorobenzne | 53.0 | 9.13 |
| 3. Phthalic anhydride | 130.8 | 8.3 |
| 4. Naphthalene | 80.2 | 7.7 |
| 5. Formaldehyde | −92 | 7.0 |
| 6. Water | 0 | 4.5 |
| 7. 1,2 Ethanediamine | 8.5 | 4.3 |
| 8. p-Xylene | 13.3 | 4.29 |
| 9. Iodosilane | −57.0 | 3.76 |
| 10. Maleic anhydride | 58.0 | 3.6 |
| 11. Chloroacetic acid | 61.2 | 3.21 |
| 12. Acetamide | 81.0 | 2.9 |
| 13. Succinimide | 125 | 2.3 |

13

# 0 044 366

TABLE 1 (contd.)

| Substance | Triple point temperature (°C) | Triple point pressure (mm Hg) |
|---|---|---|
| 14. Acrylic acid | 14 | 2.2 |
| 15. Dichloroethane | −35.3 | 2.2 |
| 16. Diacetamide | 78.5 | 2.1 |
| 17. Oxygen | −218.4 | 1.23 |
| 18. Phenol | 40.6 | 1.2 |
| 19. tert-Amyl alcohol | −11.9 | 1.1 |
| 20. l-Naphthol | 96 | 1.1 |
| Class 3: Triple point pressure less than 1 mm Hg | | |
| 1. p-Nitrotoluene | 51.9 | 0.96 |
| 2. Biphenyl | 69.5 | 0.94 |
| 3. Acetanilide | 113.5 | 0.9 |
| 4. Ethylene | −169 | 0.88 |
| 5. Silicon tetrachloride | −68.8 | 0.606 |
| 6. 2,4 Dichlorophenol | 45 | 0.59 |
| 7. 1,3 Butadiene | −108.9 | 0.49 |
| 8. Epichlohydrin | −25.6 | 0.48 |
| 9. Menthol | 42.5 | 0.4 |
| 10. Silane ($SiH_4$) | −185.0 | 0.37 |
| 11. Titanium tetrachloride | −30.0 | 0.28 |
| 12. Hydrogen peroxide | −0.9 | 0.28 |
| 13. o-Xylene | −25.2 | 0.18 |
| 14. Styrene | −30 | 0.16 |
| 15. Acrylonitrile | −82 | 0.046 |
| 16. l-Butene | −130 | 0.035 |
| 17. m-Xylene | −47.9 | 0.028 |
| 18. Ethane | −183.2 | 0.0086 |
| 19. Trichlorosilane | −126.6 | 0.003 |
| 20. Ethyl benzene | −94.9 | 0.0001 |

In the table, substances are classified into three classes: Class 1 substances having triple point pressures greater than 10 mm Hg, Class 2 substances having triple point pressures in the range of 1 mm Hg to 10 mm Hg and Class 3 substances having triple point pressures less than 1 mm Hg. It will be seen that a large volume of vapor has to be handled in these steps and the vapor volume increases as the triple point pressure of B-component decreases.

It is noted that a large volume of low pressure vapor is formed in the first zone, travels to the second zone, and is transformed into a condensed mass in the second zone. The flow of the low pressure vapor can be handled efficiently in an apparatus which has a first zone and a second zone closely spaced apart so that the vapor travels only a short distance and which also has a large cross-sectional area per unit mass flow rate. A processing unit may have many interlaid first zones and second zones. It is noted that apparatuses illustrated in Figures 12 to 17 can meet these requirements. As the operating pressure decreases, the volume flow rate increases. Therefore, the cross-sectional area per unit mass flow has to be increased and vapor travel path has to be shortened.

Some important applications of the processes of the present invention are the production ot high quality p-xylene from a mixture containing impurities such as m-xylene, o-xylene, and ethyl benzene, the production of high quality styrene from a mixture containing styrene and ethyl benzene, the production of high grade ethylene from a mixture containing ethylene and ethane, the separation of butadiene from butylene and butane, the purification of silane, and the purification of aluminium chloride.

It is important to note that many chemicals of industrial importance belong to Class 2 and Class 3 in Table 1. Operating pressures for purifying these chemicals are rather low. It is therefore important to use an apparatus which can handle such low operating pressures.

It is noted that a distillative freezing process and its modified processes work particularly well in combination with a regular distillation process. For example, a close boiling mixture may be produced in a regular distillation process and is further processed by a solid-vapor region refining operation to yield a high purity product.

**Claims**

1. A crystallization separation process for separating a multicomponent mixture which is at least partly in a liquid state and includes two key volatile components denoted respectively as A-component and B-component, the A-component not being crystallized whilst the B-component is, into a B-enriched portion and a B-lean portion through formation of a substantially pure B-solid mass, wherein the two key components form a binary system

(a) whose characteristic vapor pressure ratio defined as the ratio of the vapor pressure of A-component to that of B-component both evaluated at the tripe point temperature of B-component is in the range of 0.1 to 10 and is less than the ratio of the heat of sublimation to the heat of melting of B-component evaluated at the triple point temperature of B-component, and

(b) whose constant pressure phase diagram includes a three phase (B-enriched solid, liquid and vapor) state, a two phase (B-enriched solid and vapor) region covering a substantial concentration range above the temperature of the three phase state and a two phase (B-enriched solid and liquid) region below the temperature of the three phase state, comprising a first step of forming a vapor mixture and a first condensed mass formed of a mother liquor and a solid phase mass of substantially pure B-component by concurrently vaporizing the volatile components from the mixture in a first zone under a first temperature and a first pressure that are respectively lower than the triple point temperature and the triple point pressure of the pure B-component, the heat released in the formation of the B-enriched solid being at least partially removed by the concurrent vaporization of the two components from the mixture, and a second step of transforming, in a second zone, the vapor mixture into a second condensed mass which is at least partly in a liquid state by transferring heat therefrom while maintaining the vapor under a second pressure which is also lower than the triple point pressure of the B-component.

2. A process as claimed in claim 1, wherein the first step is carried out under substantially adiabatic conditions.

3. A process as claimed in claims 1 or 2, wherein the A-component has a greater volitility than the B-component whereby the second condensed mass is substantially liquid and is relatively enriched in A-component.

4. A process as claimed in claims 1 or 2, wherein the second condensed mass includes a solid phase which is substantially pure B-component, at least a portion of the second condensed mass being melted by transferring heat to the mass while maintaining the mass under a higher pressure than the second pressure of the second step, the higher pressure being produced by vaporizing a minor fraction of the second condensed mass and by providing pressure isolation of the zone.

5. A process as claimed in any preceding claim wherein said first step is continued until the mother liquor phase is reduced to a minor amount such that a large fraction of the A-component in the original mixture passes into the first vapor mixture thereby leaving a purified B-solid.

6. A process as claimed in claim 5, further comprising a third step of melting, in a zone, the purified B-solid produced in the first zone by pressure isolating the zone and supplying heat thereto to thereby transform a minor amount of the B-solid into vapor and to raise the zone pressure to a pressure which is higher than the triple point pressure of the B-component, the melt of the B-solid constituting a purified B-product.

7. A process as claimed in any preceding claim wherein the B-component is aluminum chloride, ferric chloride, hydrogen cyanide, hydrogen chloride, ammonia, tert-butyl alcohol, hydrogen, cyclobutane, benzene, 1,2,4,5 tetrachlorobenzene, tert-butyl chloride, 1,4 dioxane, tetrachloroethylene, chlorine, chlorine dioxide, acetic acid, 1,4 dichlorobenzene, phthalic anhydride, naphthalene, formaldehyde, water, 1,2 ethanediamine, p-xylene, iodosilane, maleic anhydride, chloroacetic acid, acetamide, succinimide, acrylic acid, dichloroethane, diacetamide, oxygen, phenol, tert-amyl alochol, l-naphthol, p-nitrotoluene, biphenyl, acetanilide, ethylene, silicon tetrachloride, 2,4 dichlorophenol, 1,3 butadiene, epichlorohydrin, menthol, silane ($SiH_4$), titanium tetrachloride, hydrogen peroxide, o-xylene, styrene, acrylonitrile, l-butene, m-xylene, ethane, trichlorosilane or ethyl benzene.

**Revendications**

1. Procédé de séparation par cristallisation destiné à séparer un mélange à plusieurs composants qui est au moins partiellement à l'état liquide et qui comprend deux composants volatils clés désignés respectivement par les termes "composant A" et "composant B", le composant A n'étant pas cristallisé tandis que le composant B l'est en une partie enrichie en B et une partie appauvrie en B par formation d'une masse solide de B sensiblement pur, dans lequel les deux composants clés forment un système binaire

a) dont le rapport caractéristique de pression de vapeur défini comme étant la rapport de la pression de vapeur de composant A et de celle du composant B évalués l'un et l'autre à la température de point triple du composant B, est compris dans l'intervalle de 0,1 à 10 et est inférieur au rapport de la chaleur de sublimation et de la chaleur de fusion du composant B évalué à la température de point triple du composant B, et

b) dont le diagramme de phase à pression constante comprend un état à trois phases (solide enrichi en B, liquide et vapeur), un domaine à deux phases (solide enrichi en B et vapeur) couvrant une plage substantielle de concentration au-dessus de la température de l'état à trois phases, et un domain à deux phases (solide enrichi en B et liquide) au-dessous de la température de l'état à trois phases, comprenant une première étape consistant à former un mélange de vapeurs et une première masse condensée formée d'une liqueur mère et d'une masse en phase solide du composant B sensiblement pur en vaporisant concurremment les composants volatils du mélange, dans un première zone, à une première température et un première pression qui sont respectivement inférieures à la température de point triple et à la pression de point triple du composant B pur, la chaleur libérée au cours de la formation du solide enrichi en B étant au moins partiellement évacuée par la vaporisation concurrente des deux composants du mélange, et une seconde étape consistant à transformer, dans une seconde zone, le mélange de vapeur en une seconde masse condensée qui est au moins partiellement dans un état liquide en transférant de la chaleur de celui-ci tout en maintenant la vapeur à une seconde pression qui est également inférieure à la pression de point triple du composant B.

2. Procédé selon la revendication 1, dans lequel la première étape est mise en oeuvre dans des conditions sensiblement adiabatiques.

3. Procédé selon la revendication 1 ou 2, dans lequel le composant A a une volatilité plus grande que le composant B, la seconde masse condensée étant sensiblement liquide et étant relativement enrichie en composant A.

4. Procédé selon la revendication 1 ou 2, dans lequel la seconde masse condensée comprend une phase solide que est du composant B sensiblement pur, au moins une partie de la seconde masse condensée étant fondue en transférant de la chaleur à la masse tout en maintenant la masse à une pression supérieure à la seconde pression de la seconde étape, la pression plus élevée étant produite par la vaporisation d'une fraction mineure de la seconde masse condensée et en établissant une isolation de la zone en ce qui concerne la pression.

5. Procédé selon l'une quelconque des revendications précédentes, dans lequel ladite première étape est poursuivie jusqu'à ce que la phase de liqueur-mère soit réduite à une faible quantité telle qu'une fraction importante du composant A contenu dans le mélange initial passe dans le premier mélange de vapeurs en laissant ainsi un solide B purifié.

6. Procédé selon la revendication 5, comprenant en outre une troisième étape consistant à faire fondre, dans une zone, le solide B purifié prodiut dans la première zone en isolant cette zone en ce qui concerne la pression et en fournissant à cette dernière de la chaleur afin de transformer ainsi une faible quantité de solide B en vapeur et d'élever la pression de cette zone jusqu'à une pression qui est supérieure à la pression de point triple du composant B, la masse fondue du solide B constituant un produit B purifié.

7. Procédé selon l'un quelconque des revendications précédentes dans lequel le composant B est du chlorure d'aluminium, du chlorure ferrique, du cyanure d'hydrogène, du chlorure d'hydrogène, de l'ammoniac, de l'alcool tert-butylique, de l'hydrogène, du cyclobutane, du benzène, du 1,2,4,5-tétrachlorobenzène, du chlorure tert-butylique, du 1,4-dioxane, du tétrachloroéthylène, du chlore, du dioxyde de chlore, de l'acide acétique, du 1,4-dichlorobenzène, de l'anhydride phthalique, du naphtalène, du formaldéhyde, de l'eau, du 1,2-éthanediamine, du p-xylène, de l'iodo silane, de l'anhydride maléique, de l'acide chloro-acétique, de l'acétamide, du succinimide, de l'acide arcylique, du dichloroéthane, du diacétamide, de l'oxygène, du phénol, de l'alcool tert-amylique, du I-naphtol, du p-nitrotoluène, du biphényl, de l'acétanilide, de l'éthylène, du tétrachlorure de silicium, du 2,4-dichlorophénol, du 1,3-butadiène, de l'épi-chlorhydrine, du menthol, du silane (SiH₄), du tétrachlorure de titane, du peroxyde d'hydrogène, de l'o-xylène, du styrène, de acrylonitrile, du l'butène, du m-xylène, de l'éthane, du tri-chlorosilane ou de l'éthyl benzène.

**Patentansprüche**

1. Kristallisationstrennverfahren zur Trennung eines Mehr-komponentengemisches, das sich mindestens teilweise in einem flüssigen Zustand befindet und zwei als A-Komponente bzw. B-Komponente bezeichnete Ausgangskomponenten enthält, von denen die A-Komponente nicht kristallisiert wird, während die B-Komponente durch Bildung einer im wesentlichen reinen festen B-Masse in einen B-angereicherten Teil und in einen B-mageren Teil überführt wird, dadurch gekennzeichnet, daß die beiden Ausgangskomponenten ein binäres System bilden,

a) dessen charakteristisches Dampfdruckverhältnis, definiert als das Verhältnis des jeweils bei der Tripelpunkttemperatur der B-Komponente ermittelten Dampfdruckes der A-Komponente zu dem B-Komponente, einen Wert im Bereich von 0,1 bis 10 hat und kleiner ist als das bei der Tripelpunkttemperatur der B-Komponente ermittelte Verhältnis der Sublimationswärme zur Schmelzwärme der B-Komponente und

b) dessen Phasendiagramm bei konstantem Druck einen Dreiphasenzustand (B-angereicherter Feststoff, Flüssigkeit und Dampf), einen Zweiphasenbereich (B-angereicherter Feststoff und Dampf), der sich über einen wesentlichen Konzentrationsbereich oberhalb der Temperatur des Dreiphasenzustandes erstreckt, und einen Zweiphasenbereich (B-angereicherter Feststoff und Flüssigkeit) unterhalb der Temperatur des Dreiphasenzustandes einschließt, und ferner gekennzeichnet durch einen ersten Schritt, bei dem ein Dampfgemisch und eine eine Mutterlauge und eine aus der im wesentlichen reinen B-Komponente bestehende feste Masse umfassende erste kon-densierte Masse durch gleichzeitiges Ver-dampfen der flüchtigen Bestandteile aus der Mischung in einer ersten Zone bei einer ersten Temperatur und einem ersten Druck gebildet werden, die jeweils niedriger als die Tripelpunkttemperatur bzw. der Tripelpunktdruck der reinen B-Komponente sind, wobei die bei der Bildung des B-angereicherten Feststoffes freiwerdende Wärme mindestens teilweise durch die gleichzeitige Verdampfung der beiden Komponenten aus der Mischung abgeführt wird, und einen zweiten Schritt, bei dem in einer zweiten Zone das Dampfgemisch in eine mindestens teilweise einen flüssigen Zustand einnehmende zweite kondensierte Masse umgeformt wird, indem Wärme abgeführt wird, während gleichzeitig der Dampf auf einem zweiten Druck gehalten wird, der ebenfalls niedriger als der Tripelpunktdruck der B-Komponente ist.

2. Verfahren nach Anspruch 1, dadurch gekennzeichnet, daß der erste Schritt bei im wesentlichen adiabatischen Bedingungen ausgeführt wird.

3. Verfahren nach Anspruch 1 oder 2, dadurch gekennzeichnet, die A-Komponente eine köhere Flüchtigkeit besitzt als die B-Komponente, wodurch die zweite kondensierte Masse im wesentlichen flüssig und relativ reich an A-Komponente ist.

4. Verfahren nach Anspruch 1 oder 2, dadurch gekennzeichnet, daß die zweite kondensierte Masse eine feste Phase umfaßt, die im wesentlichen aus reiner B-Komponente besteht, wobei mindestens ein Teil der zweiten kondensierten Masse durch Zufuhr von Wärme zu der Masse geschmolzen wird, während die Masse unter einem Druck gehalten wird, der höher als der zweite Druck des zweiten Schrittes ist und wobei der höhere Druck durch Verdampfung eines geringen Teiles der zweiten kondensierten Masse und durch eine Druckisolierung der Zone erzeugt wird.

5. Verfahren nach einem vorhergehenden Ansprüche, dadurch gekennzeichnet, daß der erste Schritt weitergeführt wird, bis die Mutterlaugenphase auf einen geringen Anteil reduziert ist, so daß ein großer Anteil an A-Komponente in der ursprünglichen Mischung in die erste Dampfmischung übergeht und dabei gereinigten B-Feststoff zurückläßt.

6. Verfahren nach Anspruch 5, gekennzeichnet durch einen dritten Schritt, bei dem der in der ersten Zone erzeugte gereinigte B-Feststoff in einer Zone geschmolzen wird, indem die Zone druckisoliert und ihr Wärme zugeführt wird, um dadurch eine geringe Menge B-Feststoffes in Dampf unzuwandeln und den Zonendruck bis auf einen Wert zu erhöhen, der über dem Tripelpunktdruck der B-Komponente liegt, wobei die Schmelze des B-Feststoffes ein gereinigtes B-Produkt darstellte.

7. Verfahren nach einem der vorhergehenden Ansprüche, dadurch gekennzeichnet, daß die B-Komponente Aluminiumchlorid, Eisenchlorid, Blausäure, Salzsäure, Ammoniak, tert.-Butylalkohol, Wasserstoff, Cyclobutan, Benzol, 1.2.4.5-Tetrachlorbenzol, tert.-Butylchlorid, 1.4-Dioxan, Tetrachloräthylen, Chlor, Chlordioxid, Essigsäure, 1.4-Dichlorbenzol, Phthalsäureanhydrid Naphthalin, Formaldehyd, Wasser, 1.2 Athandiamin, p-Xylol, Jodsilan, Maleinsäureanhydrid, Chloressigsäure, Acet-amid, Succinimid, Acrylsäure, Dichlor-äthan, Diacetamid, Sauerstoff, Phenol, tert.-Pentylalkohol, I-Naphthol, p-Nitrotoluol, Biphenyl, Acetanilid, Äthylen, Siliziumtetrachlorid, 2.4-Dichlorphenol, 1.3-Butadien, Epichlorhydrin, Menthol, Silan (SiH$_4$), Titantetrachlorid, Wasserstoffperoxid, o-Xylol, Styrol, Acrylnitril, l-Buten, m-Xylol, Äthan, Trichlorsilan oder äthylbenzol ist.

$a_A < a_B$

Fig. 1

Fig. 3

Fig. 4

$a_A > a_B$

Fig. 2

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

4

Fig. 18 — Type 1

Fig. 19 — Type 2

Fig. 20 — Type 3

Fig. 21 — Type 4

Fig. 22 — Type 5

5